# DEMANDE DE BREVET EUROPEEN

(11) **EP 0 741 296 A1**
(43) Date de publication de la demande: **06.11.1996**
(21) Numéro de dépôt: 95440025.5
(22) Date de dépôt: 05.05.1995
(51) Int. Cl.: G01N 33/80, G01N 33/52

(54) **Dispositif de carte de contrôle prétransfusionnel pour déterminer la compatibilité du sang du donneur avec le sang du receveur**

(71) Demandeur: INSTITUT JACQUES BOY, F-51065 Reims Cédex (FR)
(72) Inventeur: Menu, Marc, 51100 Reims (FR); De Beaumont, Chantal, 51100 Reims (FR); Pigani, Christophe, 21100 Dijon (FR)
(74) Mandataire: Mayran, Ninon Avocat

(57) **Abrégé**

Dispositif de carte de contrôle prétransfusionnel permettant de déterminer avant une transfusion sanguine la compatibilité du sang du donneur avec le sang du receveur .

Il comprend plusieurs volets (1, 2, 3), dont l'un (3) des volets extrêmes est un film transparent adhésif (30) dont la partie adhésive est recouverte d'une feuille de protection pelable (31), ledit film adhésif (30) étant destiné à être replié et collé sur le volet adjacent (2) sur lequel auront été réalisés préalablement, en des emplacements prévus à cet effet, les différents mélanges du sang du donneur et du sang du receveur avec les différents réactifs.

## Description

La présente invention a pour objet un dispositif de carte de contrôle prétransfusionnel permettant de déterminer avant une transfusion sanguine la compatibilité du sang du donneur avec le sang du receveur.

A ce jour les tests permettant de déterminer la compatibilité du sang d'un donneur avec celui d'un receveur sont réalisés sur des supports divers qui n'offrent pas la possibilité de conserver et/ou de fixer les résultats obtenus.

On connaît déjà des dispositifs de cartes comportant des zones pré-imprégnées de sérums-tests sur lesquelles on dépose une goutte de sang du receveur et une goutte de sang du donneur, le résultat obtenu permettant de déterminer si les deux sangs sont compatibles.

Ces cartes sont généralement réalisées en carton, et lors du mouillage des réactifs desséchés, mouillage qui nécessite une bonne homogénéisation, on peut créer localement une pâte incorporant des particules de papier, ce qui ne facilite pas la lecture du résultat et peut entrainer des erreurs d'interprétation.

De plus, les dispositifs connus présentent l'inconvénient de ne pas permettre de conserver le résultat obtenu dans son intégrité et en toute sécurité.

La présente invention a pour but de remédier à ces divers inconvénients des dispositifs connus en proposant un dispositif de carte de contrôle prétransfusionnel, qui permet de conserver l'image des différentes réactions et de les fixer de manière fiable, autorisant ainsi son archivage en toute sécurité.

Le dispositif objet de la présente invention se caractérise essentiellement en ce qu'il comprend une carte à plusieurs volets, dont l'un des volets extrêmes est un film transparent adhésif dont la partie adhésive est recouverte d'une feuille de protection pelable, ledit film adhésif étant destiné à être replié et collé sur le volet adjacent sur lequel auront été réalisés préalablement, en des emplacements prévus à cet effet, les différents mélanges du sang du donneur et du sang du receveur avec les différents réactifs.

Selon une caractéristique additionnelle du dispositif selon l'invention la carte est réalisée en un matériau imperméable et imprimable tel qu'un film multicouches de polypropylène extrudé et étiré.

Selon une autre caractéristique additionnelle du dispositif selon l'invention, le volet adjacent au volet adhésif comporte des cases imprimées agencées en lignes et en colonnes destinées à recevoir les gouttes de sang et dans certaines desquelles sera réalisé un mélange avec un réactif.

Selon une autre caractéristique additionnelle du dispositif selon l'invention, les cases imprimées sont pré-imprégnées de réactifs desséchés.

Selon une autre caractéristique additionnelle du dispositif selon l'invention, la feuille protectrice pelable comporte le mode d'emploi de la carte.

Les avantages et les caractéristiques de la présente invention ressortiront plus clairement de la description qui suit et qui se rapporte au dessin annexé, lequel en représente un mode de réalisation non limitatif.

Dans le dessin annexé :
- la figure 1 représente une vue en perspective d'un dispositif de carte selon l'invention.
- la figure 2 représente une vue en perspective du même dispositif après utilisation.

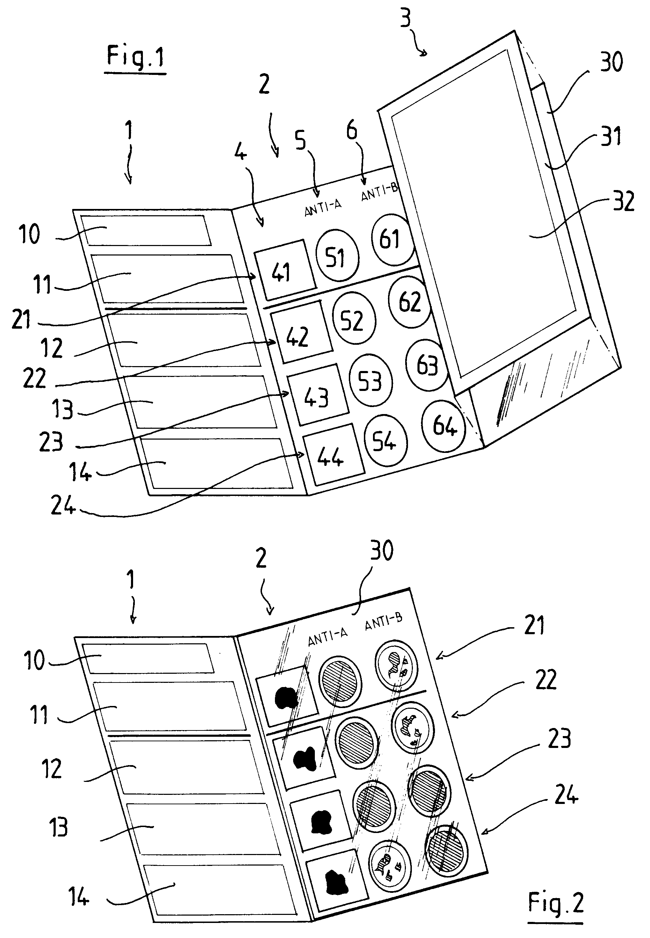

Si on se réfère à la figure 1 on peut voir qu'un dispositif de carte de contrôle prétransfusionnel selon l'invention comprend trois volets 1, 2 et 3.

Sur le volet central 2 sont imprimées des cases agencées, de façon non limitative, en trois colonnes 4, 5 et 6 et en quatre lignes 21, 22, 23 et 24.

Sur le volet 1 sont imprimées des zones 11, 12, 13 et 14 chacune en regard des lignes respectivement 21, 22, 23 et 24, destinées à l'inscription des références d'identification du receveur pour la zone 11, et du ou des différents donneurs pour les zones 12, 13 et 14, ces références pouvant se présenter sous forme d'étiquettes avec code à barres.

Le volet 1 comporte également une zone 10 destinée à l'inscription des références d'identification de l'opérateur.

Le volet 3 est constitué d'un film transparent adhésif 30 recouvert d'une feuille protectrice 31 pelable sur laquelle est imprimé le mode d'emploi 32 de la carte, l'interprétation des résultats étant imprimée au verso, non visible sur la figure, du volet central 2.

Le film transparent adhésif 30 peut être un film d'une largeur double de celle du volet central 2, dont une moitié est collée sur le verso de celui-ci, et dont l'autre moitié est recouverte de la feuille protectrice pelable 31.

Les cases 41, 42, 43 et 43 de la colonne 4 sont destinées à recevoir chacune une goutte de sang, du receveur pour la case 41, des donneurs pour les cases 42, 43 et 44,

Les cases 51, 52, 53 et 54 de la colonne 5 sont destinées à la réalisation du mélange d'une partie de la goutte de sang des cases de la colonne 4 avec un réactif anti-A, tandis que les cases 61, 62, 63 et 64 de la colonne 4 sont destinées à la réalisation du mélange d'une partie de la goutte de sang des mêmes cases avec un réactif anti-B.

Les différents mélanges peuvent être réalisés soit en prélevant les réactifs dans des flacons, soit en mouillant avec de l'eau physiologique les cases qui auront été, de fabrication, pré-imprégnées de réactifs desséchés.

Après séchage, la feuille protectrice 31 est enlevée et le film adhésif 30 est replié et collé sur le volet 2, comme représenté sur la figure 2, les résultats du test pouvant ainsi être conservés dans le dossier du patient sans altération possible.

Le matériau utilisé pour la fabrication de la carte est de préférence un film multicouches de polypropylène contenant des charges inorganiques et présentant avantageusement les qualités d'imperméabilité nécessaires à la bonne réalisation des mélanges.

## Revendications

1. Dispositif de carte de contrôle prétransfusionnel permettant de déterminer avant une transfusion sanguine la compatibilité du sang du donneur avec le sang du receveur, caractérisé en ce qu'il comprend plusieurs volets (1, 2, 3), dont l'un (3) des volets extrêmes est un film transparent adhésif (30) dont la partie adhésive est recouverte d'une feuille de protection pelable (31), ledit film adhésif (30) étant destiné à être replié et collé sur le volet adjacent (2) sur lequel auront été réalisés préalablement, en des emplacements prévus à cet effet, les différents mélanges du sang du donneur et du sang du receveur avec les différents réactifs.

2. Dispositif selon la revendication 1 caractérisé en ce que la carte est réalisée en un matériau imperméable et imprimable tel qu'un film multicouches de polypropylène extrudé et étire.

3. Dispositif selon la revendication 1 ou la revendication 2 caractérisé en ce que le volet (2) adjacent au volet adhésif (3) comporte des cases imprimées (41, 42, 43, 44 ; 51, 52, 53, 54 ; 61, 62, 63, 64), agencées en lignes (21, 22, 23, 24) et en colonnes (4, 5, 6), destinées à recevoir les gouttes de sang et dans certaines desquelles sera réalisé un mélange avec un réactif.

4. Dispositif selon l'une quelconque des revendications précédentes, caractérisé en ce que les cases imprimées sont imprégnées de réactifs desséchés.

5. Dispositif selon l'une quelconque des revendications précédentes caractérisé en ce que la feuille protectrice pelable (31) comporte le mode d'emploi (32) de la carte.
